# EUROPEAN PATENT SPECIFICATION

(11) **EP 3 947 648 B1**
(45) Date of publication and mention of the grant of the patent: **15.11.2023**
(21) Application number: 20715377.6
(22) Date of filing: 02.04.2020
(51) Int. Cl.: C12N 5/0786, A61K 35/15, A61P 19/02, A61P 21/00, C12N 5/00

(54) **COMPOSITION FOR TISSUE REGENERATION, METHOD OF PRODUCTION AND USES THEREOF**
GEWEBESPEZIFISCHE WACHSTUMSFAKTOREN, VERFAHREN ZUR HERSTELLUNG UND VERWENDUNGEN DAVON
FACTEURS DE CROISSANCE SPÉCIFIQUES À DES TISSUS, LEUR PROCÉDÉ DE PRODUCTION ET LEURS UTILISATIONS

(30) Priority: 03.04.2019 EP 19382247
(43) Date of publication of application: 09.02.2022
(73) Proprietor: Peaches S.L., 28050 Madrid (ES)
(72) Inventor: LAPUENTE FERNÁNDEZ, Juan Pedro, 28050 Madrid (ES); DE GREGORIO, Juan Carlos, 28050 Madrid (ES); DESPORTES BIELSA, Paula, 28050 Madrid (ES); FERNÁNDEZ VICENTE, Pablo, 28050 Madrid (ES); CAYUELA CALVO, María, 28050 Madrid (ES); BLÁZQUEZ MARTÍNEZ, Alejandro, 28050 Madrid (ES); DOS ANJOS VILABOA, Severiano, 28050 Madrid (ES); IGLESIAS MORENO, Carmen, 28050 Madrid (ES); GÓMEZ CEPA, Gonzalo, 28050 Madrid (ES); SANZ MORENO, Jara, 28050 Madrid (ES)
(74) Representative: Clarke, Modet y Cía., S.L.
(86) International application number: PCT/EP2020/059365
(87) International publication number: WO 2020/201404

(56) References cited:
- WO-A1-2014/149301
- TW-B- I 640 631
- DIANA TA PLOEGER ET AL: "Cell plasticity in wound healing: paracrine factors of M1/ M2 polarized macrophages influence the phenotypical state of dermal fibroblasts", CELL COMMUNICATION AND SIGNALING, BIOMED CENTRAL, LONDON, GB, vol. 11, no. 1, 19 April 2013 (2013-04-19) , page 29, XP021155505, ISSN: 1478-811X, DOI: 10.1186/1478-811X-11-29
- ALEKSEEVA O YU ET AL: "Phenotype and Secretome of Monocyte-Derived Macrophages Interacting with Mesenchymal Stromal Cells under Conditions of Hypoxic Stress", BULLETIN OF EXPERIMENTAL BIOLOGY AND MEDICINE, SPRINGER NEW YORK LLC, US, vol. 168, no. 1, 1 November 2019 (2019-11-01), pages 125-131, XP037072661, ISSN: 0007-4888, DOI: 10.1007/S10517-019-04662-2 [retrieved on 2019-11-27]
- FUNAYAMA H ET AL: "Human monocyte- endothelial cell interaction induces platelet-derived growth factor expression", CARDIOVASCULAR RESEARCH, OXFORD UNIVERSITY PRESS, GB, vol. 37, no. 1, 1 January 1998 (1998-01-01), pages 216-224, XP002118702, ISSN: 0008-6363, DOI: 10.1016/S0008-6363(97)00224-1
- LE ADRIAN D ET AL: "Current Clinical Recommendations for Use of Platelet-Rich Plasma", CURRENT REVIEWS IN MUSCULOSKELETAL MEDICINE, HUMANA PRESS, INC, US, vol. 11, no. 4, 23 October 2018 (2018-10-23), pages 624-634, XP036630108, ISSN: 1935-973X, DOI: 10.1007/S12178-018-9527-7 [retrieved on 2018-10-23]
- C. BORSELLI ET AL: "Regenerative Medicine Special Feature: Functional muscle regeneration with combined delivery of angiogenesis and myogenesis factors", PROCEEDINGS OF THE NATIONAL ACADEMY OF SCIENCES, vol. 107, no. 8, 23 February 2010 (2010-02-23), pages 3287-3292, XP055066525, ISSN: 0027-8424, DOI: 10.1073/pnas.0903875106

## Description

### FIELD OF THE INVENTION

The present invention relates to production of tissue-specific growth factors and/or cytokines. The present invention also relates to compositions comprising said growth factors and/or cytokines obtained from said method. The invention belongs to the field of human or animal medicine, in particular to the tissue regeneration.

### BACKGROUND OF THE INVENTION

Growth factors (GF) play an important role in promoting cellular differentiation and cell division. They are substances active in stimulating the growth of cells and tissues and they can be produced by many different types of tissues. Examples of these substances include insulin-like growth factors (somatomedins), which stimulate growth by mediating the secretion of growth hormone from the pituitary gland; epidermal growth factor, which stimulates the growth of epithelial cells; platelet-derived growth factor, which stimulates the growth of muscle cells and connective tissue cells; and nerve growth factor, which stimulates the growth of neuronal cells.

GF can originate from platelets, plasma, the bone matrix, osteocytes and osteoblasts, fibroblasts, bone marrow, and virtually from every tissue. It has been shown that GF from blood samples have important biological properties, such as tissue regeneration and decrease of diseases (Civinini A et al., 2010. Clin Cases Miner Bone Metab 7(3):194).

GF from blood samples generally are used in form of platelet-rich plasma (PRP), i.e. of the portion of plasma that, after centrifugation, is rich in platelets. PRP comprises growth factors and cytokines such as platelet-derived growth factor, transforming growth factor beta, fibroblast growth factor, insulin-like growth factor 1, insulin-like growth factor 2, vascular endothelial growth factor, epidermal growth factor, Interleukin 8, keratinocyte growth factor and connective tissue growth factor. The efficacy of growth factors in healing injuries, and the concentrations of the GF found within PRP are the theoretical basis for the use of PRP in tissue repair. The platelets collected in PRP are activated by the addition of thrombin and calcium chloride.

For many years, the PRP comprising grow factors and cytokines have been used in the treatment of sport injuries. This have led to different conclusions regarding the efficacy and efficiency in the treatment of injuries in different tissues, since the results have shown high variability, usually is associated with the patient's age. It has been disclosed that the growing factors concentration in PRP significantly decreases in subjects over 25 years old in healthy population, especially in men, (Evanson JR et al, 2014. Mil Med 179(7): 799). Furthermore, any disease of a subject may additionally alter the concentration of GF in PRP, key in the regeneration-recuperation process of a sport injury in soft or osteocartilaginous tissue.

All medical products in the prior art comprising GF and directed to tissue regeneration are autologous, this is, the same individual is the cell donor and the recipient. One of the more relevant features of these compositions is that they must be applied into the patient immediately after their preparation, in order to avoid losing the biological properties of the GF. During the storage of these medical products there may be changes in the GF's properties, such as lowering pH, increase of the potassium level in plasma and changes in their metabolism, which affect the biological effect of the GF (Martinez MC et al. Transfusion medical applications and practice. 2012. First edition. Cortés A. Vol I, chapter 4, p. 71).

Moreover, since blood compounds cannot survive at room temperature for long periods of time, it is recommended to apply compositions comprising thereof immediately. The recommended period between the patient's blood extraction and the PRP administration should be of no more than 45 minutes (Moreno R et al., 2015. Farm Hosp 39(3):130). A plasma supernatant must be used within eight hours from its preparation in order to avoid degradation or denaturalization of the growing factors and also avoid bacterial contamination. All the process is performed in sterile and aseptic conditions, since the composition comprising the PRP will be introduced in the subject's body.

In order to administer the GF to a patient, recent medical applications of autologous blood growth factors require infiltration or continuous administration of the product with a short interval between doses, in order the product may be effective. For these applications, the preparation of the composition comprising autologous blood compounds, requires continuous blood extraction from the patient, since fresh compositions must be prepared each time the composition is needed. This affects negatively to the patient's life quality and makes this procedure unsuitable in long-term treatments for chronic or degenerative conditions. Therefore, it would be an advantage to have a composition comprising GF able to be stored while its components remain stable and biologically active over time. This would allow the storage of the product, without having to apply to the patient immediately.

In the sports field, the patient injured requires the administration of a tissue-repairing composition as soon as possible, preferably immediately after he/she has suffered the injury, in order the patient's recovery may start as soon as possible. This is especially important in high-level athletes. A composition stored would allow to treat an injury immediately after it has occurred, which would benefit the recovery prognosis and the patient's quality of life, since no additional blood extractions would be needed.

Due to the compulsory need of frequent administration of autologous PRP compositions to a patient, there are multiple chronic or degenerative pathologies which could be potentially treated with these compositions, but they have not been treated due to the continuous requirements from the patient, negatively affecting his/her quality of life. Examples of these diseases are eye pathologies, pathologies of the central nervous system, degenerative joint pathologies, rheumatic pathologies, endocrine pathologies, sports injuries and any other disease or pathology which would require the repeated administration of a blood derived composition containing GF.

Moreover, PRP compositions deriving from platelets show an additional drawback: the growth factors, cytokines and chemokines obtained from these cells show a very high variability, both quantitative and qualitative, which make difficult to obtain reproducible batches of composition. The varying GF concentration result in different biologic effects. Thus, the GF levels in each composition should be analyzed for reliable interpretation of the biological functions and standardized application of PRP (Soon et al., 2011. Korean J Lab Med 31: 212). In fact, it has been shown that other factors such as aging, diet, lifestyle, stress, illnesses, etc. negatively affect the content thereof (Bai MY et al., 2018. Scientific Reports 8:10642).

Another interesting aspect to consider in the regeneration or repair of a tissue through the use of compositions comprising growth factors, is their pleiotropism. Although it is possible to assign to each type of GF a main property, its effect on each tissue is conditioned by the presence of other components in the composition, such as other growth factors or cytokines, which may produce an effect opposite to the desire effect for an specific growth factor. The pleiotropism is due to the fact that the cellular responses to the different cytokines and growth factors are activated when said compounds activate their receptors in the target cells. Each cytokine or each factor is typically recognized by only 1 or 2 different types of receptors and the interaction is highly specific, although it can be modulated by different co-adjuvants or antagonists of other cytokines and/or factors.

Document TW I640631-B discloses the differentiation of M0-macrophages to M2-macrophages using baicalin, obtaining a conditioned media from the culture of said M2-macrophages and using it for wound healing (fibroblast proliferation and angiogenesis).

Consequently, there is therefore a need for a growth factor and/or cytokine composition suitable to be used tissue regeneration. This composition should have a growth factor and/or cytokine profile that is suitable for regenerating a specific target tissue. This composition may be stored for long periods of time, while the growth factors and/or cytokines maintain their function and biological activity. The composition should be suitable for allogenic administration and be effective for tissue regeneration.

The present invention provides a solution for this problem disclosing a process for obtaining a composition comprising growth factors and/or cytokines, the composition obtained by said method being able to be stored and biologically active for at least one year. This composition may be applied immediately after its obtention or in any other moment to a subject needed thereof.

### DESCRIPTION OF THE INVENTION

### Brief description of the invention

The present invention refers to a method for obtaining a composition for tissue regeneration, comprising the following steps:
providing M2-macrophages, wherein this step of providing M2-macrophages comprises purifying monocytes from a sample and differentiating the purified monocytes into M2-macrophages;
co-culturing the M2-macrophages with tissue-specific cells in serum free medium; and
collecting the supernatant of the co-culture,
   wherein the ratio of M2-macrophages to tissue-specific cells in the co-culture is 1:1 to 40:1

In a preferred embodiment, the ratio of M2-macrophages to tissue-specific cells in the co-culture is, preferably 1:1 to 20:1, more preferably 10:1.

In a preferred embodiment, the co-culture is carried out at a temperature of between 30 and 40°C, more preferably at 37 °C.

In a preferred embodiment, the M2-macrophages are co-cultured with the tissue-specific cells for a period of 3 to 28 days.

In a preferred embodiment, the method further comprises cryopreservation and/or lyophilization of the composition.

In a further embodiment, the method additionally comprises the step of thawing the composition that was cryopreserved or reconstituting the composition that was lyophilized.

In a preferred embodiment, the tissue-specific cells are cells corresponding to the tissue to be regenerated.

In a preferred embodiment the tissue-specific cells are selected from myocytes, skeletal muscle cells, muscle satellite cells, tenocytes, Schwann cells, dermal fibroblasts, chondrocytes, synoviocytes, osteocytes, lymphocytes, hair follicle cells, myocardia cells, lymphatic endothelial cells, cells from respiratory epithelium, mesenchymal stem cells or combinations thereof.

In a preferred embodiment, the step of co-culturing the M2-macrophages with tissue-specific cells in serum free medium is carried out in a Transwell.

In another embodiment, the invention discloses the composition for tissue regeneration obtained by a method consisting of the following steps:
- providing M2-macrophages, wherein this step of providing M2-macrophages comprises purifying monocytes from a sample and differentiating the purified monocytes into M2-macrophages;
- co-culturing the M2-macrophages with tissue-specific cells in serum free medium; and
- collecting the supernatant of the co-culture,
wherein the ratio of M2-macrophages to tissue-specific cells in the co-culture is 1:1 to 40:1,, wherein the tissue-specific cells are selected from the group comprising myocytes, skeletal muscle cells, muscle satellite cells, tenocytes, osteocytes, Schwann cells, dermal fibroblasts, chondrocytes, synoviocytes, lymphocytes, hair follicle cells, myocardia cells, cells from respiratory epithelium, lymphatic endothelial cells or mesenchymal stem cells or combinations thereof and wherein the M2-macrophages are co-cultured with the tissue-specific cells for a period of 3 to 28 days..

In another embodiment, the invention discloses a pharmaceutical composition comprising the composition of the invention.

In another embodiment, the invention discloses a composition according to the present invention, for use in a method of treatment of a disease, wherein the composition for use is administered in a tissue comprising cells of the same type as the tissue-specific cells.

In another embodiment the invention discloses a composition according to the present invention for use in a method of treatment a disease selected from tendinosis, muscle fibrillary break, tendinitis, bursitis, synovitis, , arthrosis, cartilage break, respiratory diseaseor a chondropathy, wherein the composition for use is administered in a tissue comprising cells of the same type as the tissue-specific cells.

In another embodiment, the composition for use is administered to a subject different from the subject donor of the monocytes or the tissue-specific cells.

The present invention also refers to a method for modifying the growth factor and/or cytokine composition of the culture medium in a M2-macrophage culture, comprising: providing M2-macrophages, wherein this step of providing M2-macrophages comprises purifying monocytes from a sample and differentiating the purified monocytes into M2-macrophages; and
co-culturing the M2-macrophages with tissue-specific cells in serum free medium, wherein the growth factor and/or cytokine composition of the culture medium is modified as compared to the growth factor and/or cytokine composition of a culture medium in a M2-macrophage culture without the tissue-specific cells in serum free medium, wherein the ratio of M2-macrophages to tissue-specific cells in the co-culture is 1:1 to 40:1.

### Description of the figures

The following figures illustrate the present invention and should not be construed as limiting said invention in any way.
**Figure 1****.** Absolute concentrations (pg/ml) of growth factors and cytokines in M2-macrophages conditioned medium (M2-CM) and the tissue-specific modified compositions produced by co-culturing the macrophages with specific cell types. The stars represent significantly different values compared to those obtained in the M2-CM; CON: Chondrocyte; SIN: synoviocyte; TEN: Tenocyte; OST: Osteocyte; MIO: Myocyte; MSC: Mesenchymal stem cell; LEC: Lympho-endothelial cell; SCH: Schwann.
**Figure 2****.** Averages of the concentrations displayed in Figure 1. The shaded boxes represent significantly different values compared to those obtained in the M2-CM; ND: Non-detected (concentration lower than the assay sensitivity).

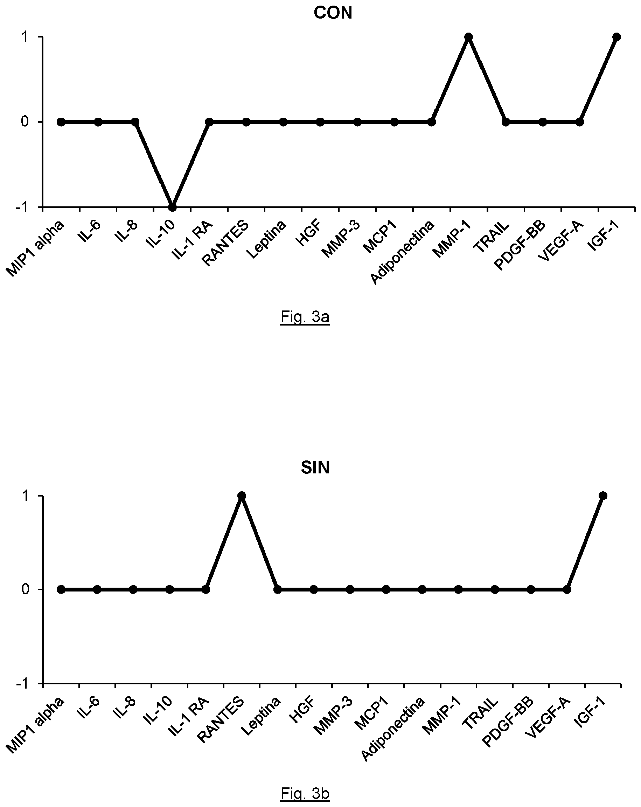

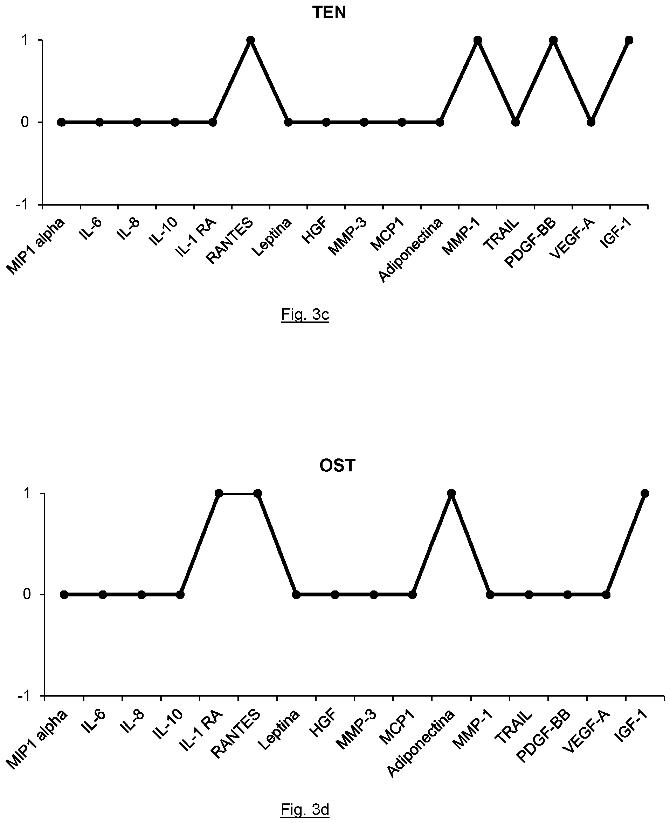

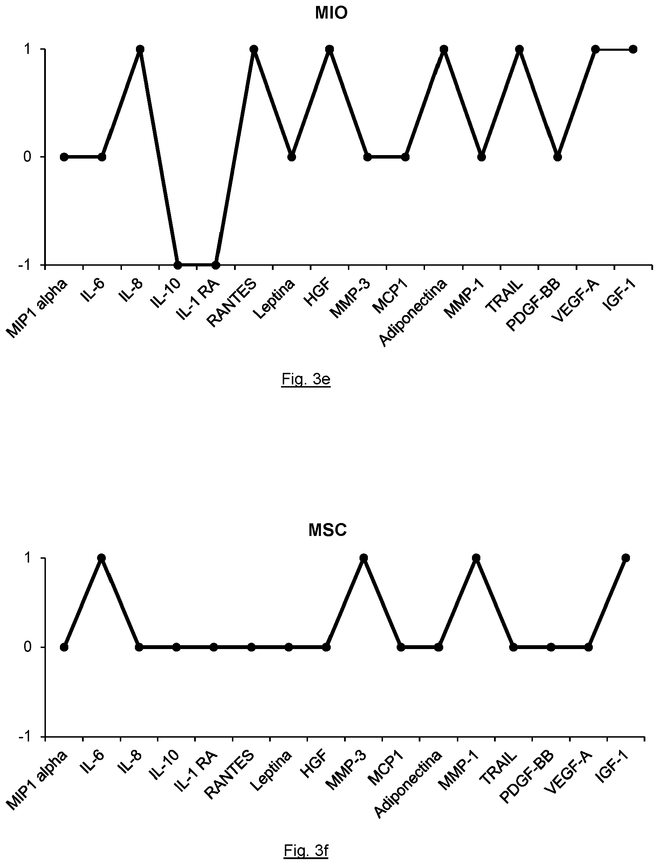

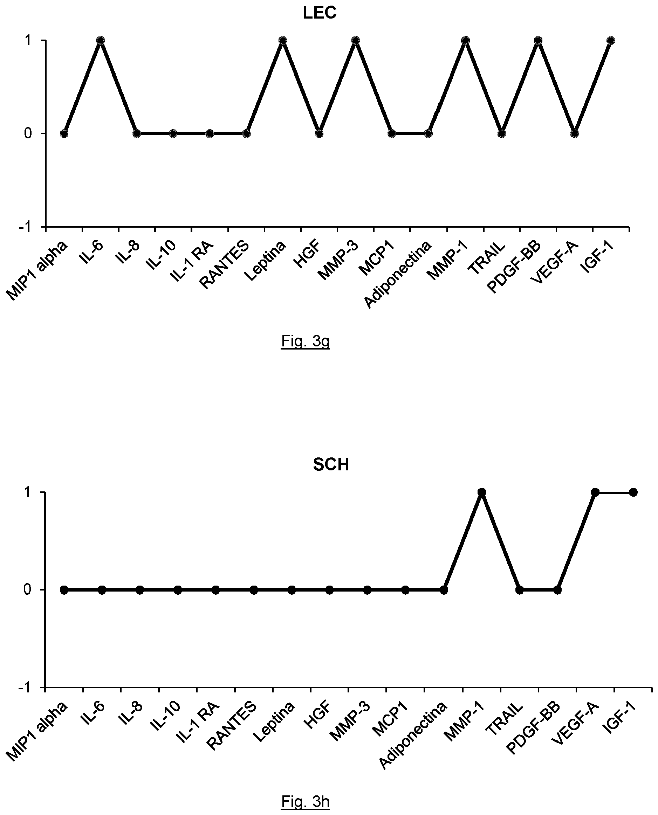

**Figure 3****.** Modification patterns of the growth factors and cytokines concentrations of the tissue-specific compositions. The tissue-specific compositions have a characteristic modification pattern, regardless of the starting concentrations in M2-CM. Central line: molecular concentration similar to macrophage M2 Conditioned Medium; Upper line: molecular concentration significantly higher than M2-CM; Bottom line: molecular concentration significantly lower than M2-CM. All statistics were calculated with data from five different patients (five different starting concentrations in M2-CM) and repeated three times for each one in independent assays. ANOVA was performed to determine statistically significant differences between the independent experimental groups studied, comparing the mean of each column with the mean of the control group (macrophages). GraphPad Prism version 8.4.0 for Mac OS X was used (GraphPad Software, San Diego, California USA). Statistical significance level was placed at P<0.05.
**Figure 4****.** Evolution of the percentage of patients suffering from patellar tendinitis of phase II (II, black) and phase III (III, white) from day 0 (control) until day 27 after treatment.
**Figure 5****.** Evolution of the percentage of patients suffering from Achilles tendinitis of phase II (II, black) and phase III (III, white) from day 0 (control) until day 30 after treatment.
**Figure 6****.** Evolution of the percentage of patients suffering from muscle ruptures of phase II (II, black) and phase III (III, white) from day 0 (control) until day 42 after treatment.
**Figure 7****.** Echography of a subject with a muscle rupture done on the day previous to the treatment (-1), the day 4 after the treatment (+4) and the day 8 after the treatment (+8).
**Figure 8****.** Evolution of the percentage of patients suffering from gonarthrosis of phase II (II, black) and phase III (III, white) from day 0 (control) until day 56 after treatment.
**Figure 9****.** Evolution of the percentage of patients suffering from chondromalacia patellae of phase II (II, black) and phase III (III, white) from day 0 (control) until day 56 after treatment.
**Figure 10****.** Evolution of the percentage of patients suffering from anterior cruciate ligament rupture of phase II (II, black) and phase III (III, white) from day 0 (control) until month 5 after treatment.
**Figure 11****.** Evolution of the percentage of patients suffering from dynamic osteopathy of the pubis of phase II (II, black), phase III (III, white) and phase IV (IV, grey) from day 0 (control) until month 4 after treatment.
**Figure 12****.** Evolution of the percentage of patients suffering from astragalus fibular ligament sprain of phase II (II, black) and phase III (III, white) from day 0 (control) until day 24 after treatment.

### Detailed description of the invention

For purposes of the present invention, the expressions "regenerative medicine", "tissue repair", "injuries in a tissue", "tissue recuperation", "tissue regeneration" or "tissue healing" should be considered synonymous of any process which implies the healing of a tissue comprised of mammal cells previously injured in any way. Preferably, the process implies the regeneration of the wounded tissue.

For the purposes of the present invention, the expressions "composition for tissue regeneration", "tissue-specific composition", or simply "composition" are all synonyms and refer to compositions produced by the method of the present invention. As is described below, examples of such compositions include the supernatant of the co-culture of M2-macrophages and tissue-specific cells, and related purified and/or concentrated compositions, lyophilized compositions, cryopreserved compositions, and pharmaceutical compositions.

The present invention refers to a method for obtaining a composition for tissue regeneration, comprising the following steps:
providing M2-macrophages, wherein this step of providing M2-macrophages comprises purifying monocytes from a sample and differentiating the purified monocytes into M2-macrophages;
co-culturing M2-macrophages and tissue-specific cells in serum free medium; and collecting the supernatant of the co-culture,
wherein the ratio of M2-macrophages to tissue-specific cells in the co-culture is 1:1 to 40:1.

According to the present invention, the M2-macrophages used in the co-culture step are provided by differentiation of monocytes.

As is known by a skilled person, monocytes differentiate into macrophages, which in turn may undergo polarization into M2-macrophages. The source of the monocytes may be a blood sample from the same or a different subject donor of the tissue-specific cells. In a preferred embodiment, the origin of the monocytes is a blood sample from the same subject donor of the tissue-specific cells. Further, the monocytes may be autologous or allogenic, such that the monocytes used for obtaining the composition for tissue regeneration may belong to a donor that is the same or a different subject that will be treated with said composition.

Monocytes may be obtained either by apheresis, from whole blood by gradient centrifugation with a Ficoll medium, or by any other technique known in the art. Apheresis is an extracorporeal medical technology in which the blood of a person is passed through an apparatus that separates out one particular constituent and returns the remainder to the circulation. Blood is filtered to remove the stem cells. Gradient Centrifugation by Ficoll medium (Ficoll-Paque i.e.) is a technique which separates blood to its components. The medium is normally placed at the bottom of a conical tube, and blood is then slowly layered above the medium. After being centrifuged, the following layers are visible in the conical tube, from top to bottom: plasma and other constituents, a layer of mono-nuclear cells called buffy coat (PBMC/MNC)-Peripheral Blood Mononucleated Cells.

Preferably, purified monocytes are used for obtaining M2-macrophages. Purification of monocytes may be performed by placing them in culture under normoxic or hypoxic conditions of total sterility. Since the composition obtained by the process of the invention will be applied in a subject, it is necessary that all the steps are performed under sterile conditions. In the body, oxygen concentrations range from 1 to 12%, rather than the 21 % in the atmosphere. Cells cultured in low oxygen, or hypoxia, grow faster, live longer and show lower stress. The oxygen concentration in hypoxic conditions is 3 - 5 %. Monocytes may be selected, for example, by adherence after a 24-hour incubation culture or by a second centrifugation in a density gradient.

Monocytes may be differentiated into M2-macrophages per methods known in the art. For example, monocytes may be cultured in serum free medium in presence of macrophage colony-stimulating factor (M-CSF), thereby inducing differentiation of the monocytes into M2-macrophages. Alternative methods for inducing differentiation are known in the art, which include using IL-4, IL-10, IL-13 and/or TGF-beta as differentiation agents. Preferably, monocytes are cultured for 3-5 days, in presence of 10 ng/ml of M-CSF. The so-produced cells may then be used for the co-culture step with the tissue-specific cells.

As will be understood by a skilled person, non-differentiated monocytes may remain in the cell culture after the differentiation process. Thus, the M2-macrophages to be used for the co-culture step may be accompanied by a number of monocytes, preferably less than 30%, more preferably less than 20% monocytes. It is very important to avoid any ingredient that may interfere with differentiation into M2-macrophages, such as bovine fetal serum, phenol red, antibiotics or antifungals among others.

For purposes of this invention, tissue-specific cells refer to one or more cells specific from a tissue. The tissue-specific cells may be selected according to the target tissue to be regenerated, so as to produce compositions for tissue regeneration that have a growth factor and/or cytokine profile that is optimal to treat the target tissue. Particularly, the tissue-specific cells may correspond to the tissue to be treated with the composition for tissue regeneration. In a preferred embodiment, the tissue-specific cells are cells selected from: myocytes, skeletal muscle cells, muscle satellite cells, tenocytes, osteocytes, Schwann cells, dermal fibroblasts, chondrocytes, synoviocytes, lymphocytes, lymphatic endothelial cells, cells from respiratory epithelium, hair follicle cells, myocardia cells, mesenchymal stem cells or combinations thereof. In a preferred embodiment, the cells are from human origin. The cells may be obtained from a cell collection, or from a primary cell culture from a tissue of a subject (donor). The tissue-specific cells may be autologous or allogenic, such that the tissue-specific cells used for obtaining the composition for tissue regeneration may belong to a donor that is the same or a different subject that will be treated with said composition. The cells of the tissue may be obtained from a healthy tissue or from a damaged tissue (due to a disease or injury).

The co-culture of the M2-macrophages and the tissue-specific cells modifies growth factor and/or cytokine production, as compared to growth factor and/or cytokine production by M2-macrophages only. During the co-culture step, growth factors and/or cytokines are secreted into the culture medium, thereby producing a conditioned medium. Therefore, growth factors and/or cytokines produced according to the present invention are present in the supernatant of the cell culture. The conditioned medium (which could be referred to as a "modified M2-macrophage conditioned medium") has a different growth factor and/or cytokine profile as compared to a composition produced by culturing M2-macrophages without the tissue-specific cells (M2-macrophage conditioned medium (M2-CM)). Within the scope of the present invention, "conditioned medium", "modified M2-macrophage conditioned medium", and "supernatant", are all synonyms and are compositions useful for tissue regeneration.

Therefore, in a further embodiment, the present invention refers to a method for modifying the growth factor and/or cytokine composition of the culture medium in a M2-macrophage culture, comprising:
providing M2-macrophages, wherein this step of providing M2-macrophages comprises purifying monocytes from a sample and differentiating the purified monocytes into M2-macrophages; and
co-culturing the M2-macrophages with tissue-specific cells in serum free medium, wherein the growth factor and/or cytokine composition of the culture medium is modified as compared to the growth factor and/or cytokine composition of a culture medium in a M2-macrophage culture without the tissue-specific cells in serum free medium, wherein the ratio of M2-macrophages to tissue-specific cells in the co-culture is 1:1 to 40:1.

According to the present invention, co-culturing the tissue-specific cells with the M2-macrophages induces production of growth factors and/or cytokines in relative quantities that are optimal for treating the target tissue. More particularly, the growth factor and/or cytokine profile of the medium that has been conditioned by co-cultured cells according to the present invention is specific to each tissue, with specific molecular patterns or signatures that optimize the natural regenerative process of the respective tissue. This allows to obtain compositions having different growth factor and/or cytokine profiles depending on the tissue-specific cells used in the co-culture. Therefore, the tissue-specific compositions obtained by the inventive method optimally treat a specific target tissue.

The co-culture of the M2-macrophages and the tissue-specific cells may further comprise adult mesenchymal cells, being from the same or different origin (cell collection or primary cell culture from the tissue of a donor) than the tissue-specific cells (autologous or allogenic adult mesenchymal cells). Likewise, the co-culture of the M2-macrophages and the tissue-specific cells may further comprise monocytes, being from the same or a different subject donor of the tissue-specific cells. Such monocytes may be non-differentiated monocytes from the culture from which the M2-macrophages were obtained.

In a preferred embodiment, the M2-macrophages are co-cultured with a primary culture of cells of a tissue which can be the same tissue in which the obtained composition will be applied in a subject.

In a preferred embodiment the co-culture step is performed between 30 and 40°C, preferably at 37 °C. The culture time may be from 3 to 28 days. The co-culture may also be carried out under hypoxic conditions. Preferably, the co-culture is carried out under sterile conditions.

Preferably, the co-culture is performed with a ratio of M2-macrophages to tissue-specific cells ranging from 1:1 to 20:1, more preferably 10:1.

The co-culture may be performed in any culture medium that is suitable for culturing monocytes and/or macrophages, provided that the culture medium is serum-free. This includes standard serum free mediums.

The collection of the supernatant may be done by any method known by a person skilled in the art. Growth factors and/or cytokines are excreted into the culture medium during the co-culture and are specific to the tissue from which the tissue-specific cells come from. Therefore, the supernatant is enriched with the secretome of the co-cultured cells. The collection of the supernatant may be carried out by separating it from the culture cells by using any method known in the art. Preferably, the collection is carried out a temperature between 5 and 40 °C, more preferably at 15 °C. These low temperatures avoid or minimize the loss of volatile constituents present in the plasma or in the culture supernatant and allow maintaining a very low risk of microbial contamination in the composition. The collected supernatant may subsequently be processed so as to purify and/or concentrate the growth factors and/or cytokines it contains. For example, cell debris may be removed by filtering with a 0,22 µm membrane, or ultra-centrifuged to obtain a concentrated composition. Such purified and/or concentrated supernatant is/are an inventive composition useful for tissue regeneration.

The method may further comprise the nanoencapsulation of the growth factors and/or cytokines obtained according to the present invention. Nanoencapsulation of therapeutic agents increases their efficacy, specificity and targeting ability. Nanocarriers protect their payload from premature degradation in the biological environment, enhance bioavailability, and prolong presence in blood and cellular uptake. In a preferred embodiment, the growth factors are encapsulated by microfluidic technology.

The composition may be further processed to allow long-term storage and subsequent use. For example, it may be cryopreserved and/or lyophilized.

Cryopreservation of the collected growth factors and/or cytokines may be carried out by freezing the composition containing them (which may be supernatant of the co-culture or a purified and/or concentrated version thereof) at a temperature between -20 and - 196 °C. In a preferred embodiment, the composition is frozen at -35 °C and then cryopreserved at a temperature between -35 °C to -86 °C. In an even more preferred embodiment, the composition is frozen at -35 °C and then submitted to a temperature freezing ramp (-1 °C/min) until -86 °C. In a most preferred embodiment, the composition is first frozen and cryopreserved at -35 °C for 24 hours and then, gradually submitted to lower temperatures until -86 °C.

Lyophilization of the collected growth factors and/or cytokines may be carried out by cooling and drying the composition containing them. Preferably, the composition is cooled at a temperature of -20 to -80 °C and a pressure of 10-500 mbars.

The cryopreserved or lyophilized composition may be stored between -80 and -86 °C until its use. Storage from -40 °C has been experimentally proved to ensure stability and biological activity of the GF and/or cytokines present in the composition and their activity *in vivo* restoring an injured tissue as well. The composition cryopreserved may be maintained at least 1 year between -20 and -196 °C. Preferably no more than 3 years. In a preferred embodiment, the supernatant is dosed in single-doses.

The composition of the invention, like any other tissue regeneration composition, should be applied at room temperature (25 - 30 °C) and/or physiologic temperatures (close to 37°C). This temperature is important, since the purpose of the composition is for use as a treatment in regenerative medicine, which is performed always at these temperatures, since the treatment is applied directly in the injured tissue of a subject.

For this reason, the thawing of the cryopreserved composition should be carried out by maintaining the composition at a temperature of between 20 to 37 °C for 5 minutes to 1 hour. In a preferred embodiment, the thawing of the cryopreserved composition is performed by maintaining it between 25 and 30 °C between 20 and 40 minutes. Similarly, the lyophilized composition may be reconstituted by rehydration and then heated to a suitable temperature prior to its application.

The composition of the invention can be used in the subject donor of the tissue-specific cells (autologous) or in a different subject (allogenic), without risk of immune reaction in the recipient.

The thawed or reconstituted composition has an analogous concentration of growth factors and/or cytokines with the same biological activity, in relation to the culture medium from which the growth factors and/or cytokines are collected during the co-culture step. Therefore, the composition obtained under the method disclosed herein is stable over the time after cryopreservation and/or lyophilization. This stability and effectivity of the composition after cryopreservation and/or lyophilization allows storage of the composition and avoid any subsequent blood extraction to the donor, since doses of the composition obtained by the process described herein may be applied subsequently according to a patient's needs (the same subject as the donor or any other recipient).

It has been also experimentally proved that the effects on cell proliferation, cell migration, chemotaxis and auto and paracrine synthesis of growth factors induced by the composition after storage are identical to those of the composition before cryopreservation or lyophilization. Therefore, the cryopreservation and/or lyophilization of the composition have no effect in the biological activity in the receptor tissue.

Impurities may be carried from the blood sample into the composition of the invention. Therefore, in an embodiment, the composition obtained by the method of the invention may contain platelets, red cells or leukocytes in an amount of less than 1 % of the total amount of components in the sample; for example, less than 0.5 %, less than 0.1%, particularly, less than 0.001 % of platelets, red cells or leukocytes of the total amount of components in the sample.

Another aspect of the invention refers to a pharmaceutical composition comprising the composition of the present inventionand a pharmaceutically acceptable carrier. As would be appreciated by one of skill in this art, carriers may be chosen based on the route of administration as described below, the location of the target issue, the drug being delivered, the time course of delivery of the drug, etc. In an embodiment, the pharmaceutically acceptable carrier is selected so as not to unduly affect the biological activity of the composition of the invention. They may include emulsifiers, salts, pharmacologically acceptable buffers, binders, glidants, lubricants, disintegrants, sweeteners, pigments, co-solvents, surfactants, oils, humectants, emollients, preservatives, stabilizers, antioxidants or combinations thereof.

The pharmaceutical composition can be administered to a subject by any means known in the art, preferably directed to the injured tissue. The term "subject" as used herein, refers to humans as well as non-humans. For instance, the non-humans may be mammals (e.g., a rodent, a mouse, a rat, a rabbit, a monkey, a dog, a cat, a primate, or a pig).

In an embodiment, the composition or pharmaceutical composition disclosed herein may be stored in an injectable sterile vial. The composition or pharmaceutical composition may be administered directly to the injured tissue. In a more preferred embodiment, the composition is administered, for example, via intra-articular, subdermal, subcutaneous, intravenous or intratendinous injection.

In an embodiment, the composition or pharmaceutical composition is a sterile cell-free physiological aqueous solution with complex compositions that contains a mix of growth factors and cytokines that show specific tissue concentration patterns to provide optimal tissue-specific regenerative effects.

In another embodiment, the invention discloses a composition of the present invention; for use in a method of treatment of a disease, wherein the composition is administered in a tissue comprising cells of the same type as the tissue-specific cells of the co-culture step..

In another embodiment, the invention discloses a composition of the present invention; for use in a method of treatment a disease selected from tendinosis, muscle fibrillary break, tendinitis, bursitis, synovitis, , arthrosis, cartilage break, pulmonary disease, heart disease or chondropathies; wherein the composition is administered in a tissue comprising cells of the same type as the tissue-specific cells of the co-culture step.

The steps of the method for obtaining the composition for use as indicated above should be interpreted as previously described for other embodiments in the present document.

The composition, or pharmaceutical composition, is for use in the treatment of a disease in needed of regenerative medicine, tissue repair, tissue regeneration or tissue healing. The disease may be any disease which requires tissue healing after having suffered any kind of traumatic event. The term "repair", when used in the context of the healing of damaged tissue, is defined as the restoration of tissue architecture and function after an injury. Certain tissues of the body are more capable of cellular proliferation (and hence regeneration) than others. Regenerative medicine is defined as the treatment of a disease which require regeneration of damaged biological tissues.

The composition described herein is for use in the treatment of tissues which have been injured and require tissue regeneration. In a preferred embodiment, the growth factors and/or cytokines present in the composition are tissue-specific. This means that they have been produced in a culture comprising M2-macrophages and, as tissue-specific cells, cells of the same type of the cells which form part of the tissue to be recovered/treated/regenerated. This allows specific treatment to a tissue with specific growth factors and/or cytokines produced by cells of the same tissue. It has been experimentally demonstrated that the tissue-specific compositions - have improved biological properties, producing better results *in vitro* and *in vivo* in tissue regeneration.

In a more preferred embodiment, he composition is for use in the treatment of a disease which belongs to the field of odontology, maxillofacial surgery, sport medicine, traumatology, anesthesiology, pain unit, dermatology, aesthetic medicine, plastic, repair and aesthetic surgery, rheumatology, neurology, ophthalmology, general medicine, endocrinology, urogynecology, neurosurgery, cardiology, pneumology, cardiovascular surgery or rehab, among others.

In a more preferred embodiment, the composition is for use in tendinosis, muscle fibrillary break, tendinitis, bursitis, synovitis, arthrosis, cartilage break, or a chondropathy.

In a more preferred embodiment, the treatment is for use in regenerative medicine.

In a more preferred embodiment, the composition, or a pharmaceutical composition for use in regenerative medicine is directed to the repair of a tissue selected from the group comprising cartilaginous tissue, bone tissue, synovial tissue, corneal tissue, nerve tissue, fatty tissue, tendon tissue, ligamentous tissue, muscle tissue, epithelial tissue, endothelial tissue, hair follicles and any other tissue or specific cell group of any part of the human or animal tissue.

In a specific embodiment, the composition for use in the repair of cartilaginous tissue has been obtaining by using as tissue-specific cells, chondrocytes and/or chondroblasts.

In another specific embodiment, the composition for use in the repair of bone tissue has been obtaining by using as tissue-specific cells, osteoblasts and/or osteocytes and/or osteoclasts.

In another specific embodiment, the composition for use in the repair of synovial tissue has been obtaining by using as tissue-specific cells, fibroblast synovial cells or type B cells and/or macrophage-like synovial cells.

In another specific embodiment, the composition for use in the repair of corneal tissue has been obtaining by using as tissue-specific cells, corneal epithelium cells and/or corneal endothelium cells.

In another specific embodiment, the composition for use in the repair of nerve tissue has been obtaining by using as tissue-specific cells, neurons and/or glia and/or Schwann cells.

In another specific embodiment, the composition for use in the repair of fatty tissue has been obtaining by using as tissue-specific cells, adipocytes.

In another specific embodiment, the composition for use in the repair of tendon tissue has been obtaining by using as tissue-specific cells, tenocytes (fibrocytes) and/or tenoblasts (fibroblasts).

In another specific embodiment, the composition for use in the repair of ligamentous tissue has been obtaining by using as tissue-specific cells, periodontal ligament stem cells and/or fibrocytes.

In another specific embodiment, the composition for use in the repair of muscle tissue has been obtaining by using as tissue-specific cells, myocytes.

The composition for use in the repair of epithelial tissue has been obtaining by using as tissue-specific cells, epithelial cells and/or fibroblasts.

In another specific embodiment, the composition for use in the repair of endothelial tissue has been obtaining by using as tissue-specific cells, endothelial cells.

The composition for use in the repair of hair follicles has been obtaining by using as tissue-specific cells, primary hair follicles and/or fibroblasts.

In another specific embodiment, the composition for use in the repair of lung tissue has been obtaining by using as tissue-specific cells, cells derived from the respiratory epithelium.

In a more preferred embodiment, the composition for use described in the previous embodiments, is directed to the treatment of patellar tendinosis, partial muscular ruptures, Achilles tendonitis, gonarthrosis, patellar chondropathy, anterior cruciate ligament tear, dynamic pubic osteopathy or sprained astragalus ligament.

Disclosed herein is a method for treatment a disease which requires tissue regeneration, comprising administering an effective pharmaceutical amount of the pharmaceutical composition described above. In a preferred embodiment, the disease is selected from the group comprising tendinosis, muscle fibrillary break, tendinitis, bursitis, synovitis, arthrosis, cartilage break, respiratory disease, cardiac disease, or chondropathies or any other disease which require regenerative medicine.

In a preferred embodiment, the composition for use according to the previous embodiments is administered to a subject different from the subject donor of the monocytes or the tissue-specific cells, this is, the composition is for allogenic use.

In an embodiment, the composition of the present invention is applied in the target tissue after completing the method of its obtention. Alternatively, in a preferred embodiment, the composition for use is cryopreserved or lyophilized after the recollection step and thawed prior to its use in the treatment of the disease. In a more preferred embodiment, the composition is thawed by maintaining the composition between 25 and 30 °C between for 10 and 60 minutes.

The composition of the present invention has proven to be effective in healing damaged tissues, reducing the time of recovering of the subject comprising the tissue damage. The main reason behind the success of the composition is the presence of specific growth factors and/or cytokines for each specific tissue. The compositions are also particularly advantageous because they maintain their biological function despite having been cryopreserved or lyophilized.

In the present disclosure, the technical and scientific terms used in the descriptions herein will have the meanings commonly understood by one of ordinary skill in the art, unless specifically defined otherwise.

### EXAMPLES

The examples described below have as an object to illustrate this invention, but without limiting the scope thereof.

### Example 1: Preparation of the composition

Bags of monocytic apheresis (N=5 patients) were centrifuged in a density gradient with Ficoll 30 minutes at 400 xg. The intermediate band of peripheral blood mononuclear cells (PBMC) was collected. The fraction was washed four times with balanced salt solution. The cells were resuspended in culture medium for cell counting using the TC-20 Cell Counter device (Biorad).

The cells obtained were cultured in T175 plates for 24 hours at 37 °C and the monocytes were selected by adherence to the culture plate.

The monocytes were extracted from the gradient fraction and cultured in GMP medium (GIBCO^{®} CTS^{™} AIM V^{®} SFM, Thermofisher) without phenol red and without antibiotics. 100 µL of Dipeptiven was added per 100 ml of culture medium (1 µL/mL).

The monocytes were differentiated into M2-macrophages by adding 10 ng/ml of M-CSF and culturing them for four days.

Primary cultures of tissue-specific cells, such as human chondrocytes, human synoviocytes, human Schwann cells, human skeletal muscle cells, human osteoblasts and lymphatic endothelial cells were provided by Innoprot (Derio, Spain). Human tenocytes were obtained from Zen-Bio (NC, USA) and the human mesenchymal stem cells come from primary cultures obtained from samples of abdominal fat tissue.

The tissue-specific cells were cultured at 37 °C in poly-lysine pre-treated culture flasks in culture media as described below until obtaining enough cell numbers for the experiments:
- Human tenocytes: 100 mL alpha-MEM, 240 µL heparin, 5 mL lysed platelets, 1 mL antibiotics (penicillin-streptomycin), 1 mL glutamine, 0.5 mL amphotericin B.
- Mesenchymal stem cells: 100 mL alpha-MEM, 240 µL heparin, 5 mL lysed platelets, 1 mL antibiotics (penicillin-streptomycin), 1 mL glutamine, 0.5 mL amphotericin B.
- Human skeletal muscle cells (miocytes): 100 mL alpha-MEM, 240 µL heparin, 5 mL lysed platelets, 1 mL Skeletal Muscle Cell Growth Supplement (SKMCGS, Innoprot Ref: P10975), 1 mL antibiotics (penicillin-streptomycin), 1 mL glutamine, 0.5 mL amphotericin B.
- Human chondrocytes: 100 mL alpha-MEM, 240 µL heparin, 5 mL lysed platelets, 1 mL CGS (Chondrocyte Growth Supplement, Innoprot Ref: P10970), 1 mL antibiotics (penicillin-streptomycin), 1 mL glutamine, 0.5 mL amphotericin B.
- Human Schwann cells: 100 mL alpha-MEM, 240 µL heparin, 5 mL lysed platelets, 1 mL SCGS (Schwann Cells Growth Supplement, Innoprot Ref: P10351), 1 mL antibiotics (penicillin-streptomycin), 1 mL glutamine, 0.5 mL amphotericin B.
- Human synoviocytes: 100 mL alpha-MEM, 240 µL heparin, 5 mL lysed platelets, 1 mL SGS (Synoviocyte Growth Supplement, Innoprot Ref: P10972), 1 mL antibiotics.
- Human Lymphatic Endothelial Cells (LEC): 100 mL alpha-MEM, 240 µL heparin, 5 mL lysed platelets, 1 ml ECGS (Endothelial Cell Growth Supplement, Innoprot Ref: ECGS),1 mL antibiotics (penicillin-streptomycin), 1 mL glutamine, 0.5 mL amphotericin B.
- Human Osteoblasts: 100 mL alpha-MEM, 240 µL heparin, 5 mL lysed platelets, 1 ml OGS (Osteoblast Growth Supplement, Innoprot Ref: P10979), 1 mL antibiotics (penicillin-streptomycin), 1 mL glutamine, 0.5 mL amphotericin B.

The tissue-specific cells were then co-cultured with the M2-macrophages. The co-culture of macrophages with tissue-specific cells was performed using Transwell Inserts (Corning) using a specific ratio of M2-macrophages to Tissue Specific Cells followed by incubation at 37°C and 5 % CO2 in Gibco serum-free medium. Each co-culture and experimental condition was assayed in duplicate or triplicate.

More specifically, for 6-well culture plates 2 million monocytes/macrophages were seeded per insert (750.000 for 12-well plates). For the tissue specific cells in each co-culture between 50.000 and 100.000 cells were seeded per well (25.000-50.000 in 12 well culture plates). Total volume per well used varied from 2,25 (12-well) to 3,5 ml (6-well). Culture wells were then incubated at 37°C and 5 % CO2 for a minimum of 4 days and a maximum of 28 days. Culture supernatants were obtained every 3-4 days and culture medium changed by fresh medium each time. The collected medium containing growth factors and cytokines was then centrifuged at 3000 rpm for 10 minutes and the supernatant stored at -80°C for further analysis.

### Example 2: Analysis of the composition

When the tissue-specific cells were previously frozen, thawing was carried out by seeding the cells in T75 plates with PLL and adding 20 mL of culture media, except for human tenocytes and mesenchymal stem cells, which were centrifuged at 400 g for 7 minutes prior to adding the cells to the culture media. Different internal controls were used in each experiment: monocytes from each patient alone and tissue specific cells alone.

The cytokines and growth factors in the compositions obtained from 5 different patients using 8 different tissue-specific cells were quantified by Multiplex analysis using Multiplex Technology (Procarta Plex23 (PPX-23), Merck Millipore) and also individually by means of ELISA kits (human IGF-1, Ref EH0165, Fine test; Human TGF-beta 3, Ref EH0289 Fine Test; Human BMP-6, Ref.EHBMP6; Human VEGF-C, Ref BMS297-2; Human TGF beta 1, Ref BMS249-4; Human IL-1B, Ref BMS224-2; Human IL-4, Ref BMS225-2) . The following compounds were all analyzed together in the Multiplex Assay:, the platelet-derived growth factor BB (PdGF BB), , the vascular endothelial growth factor A (VEGF-A,), the epidermal growth factor (EGF), the interleukin 2 (IL 2), the interleukin 6 (IL 6), the interleukin 8 (IL 8), the interleukin 10 (IL 10), the of interleukin IL-12p70 (IL 12p70), the adiponectin, the Fibroblast Growth Factor2 (FGF-2), GM-CSF, HGF, IL-1RA, Leptin, MCP-1, MIP-1 alpha, MMP-1, MMP-3, NGF Beta, RANTES, TIMP-1, TRAIL and the tumor necrosis factor alpha (TNF alpha). We have followed the protocol according to manufacturer's instructions.

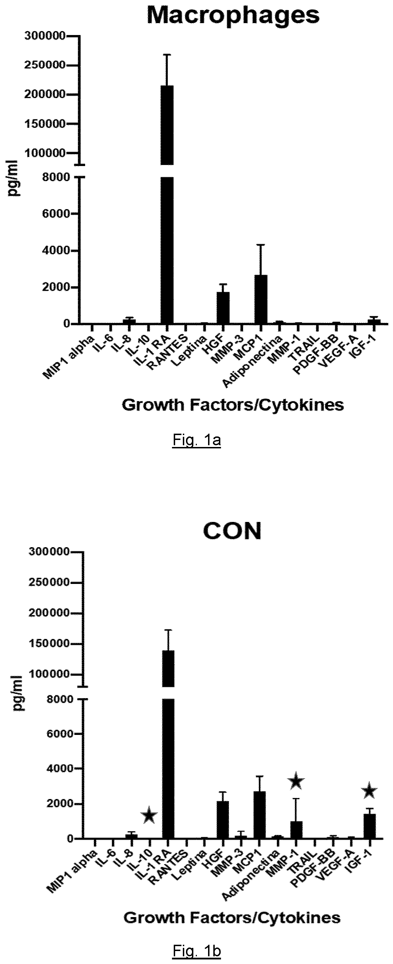

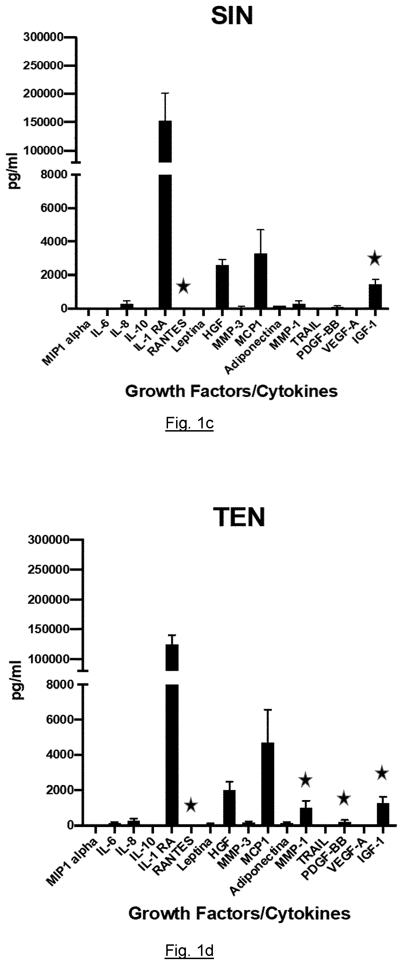

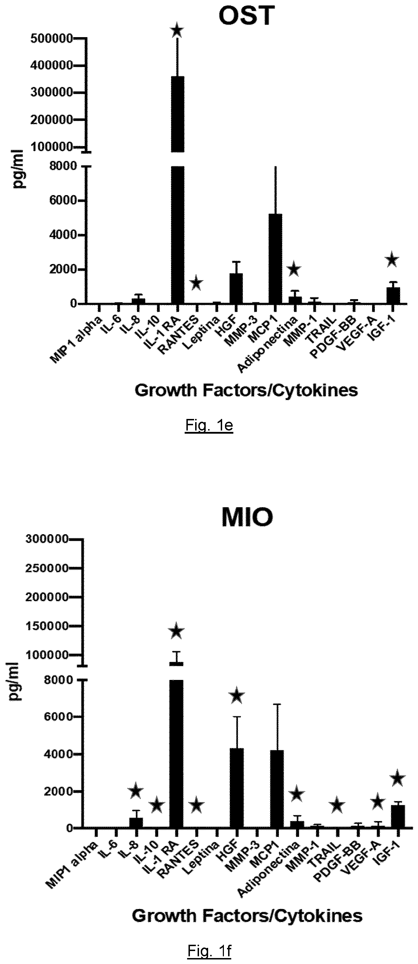

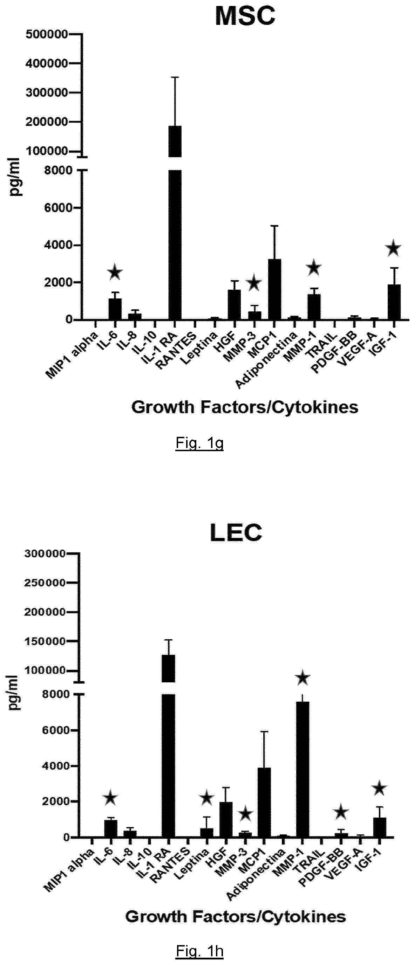

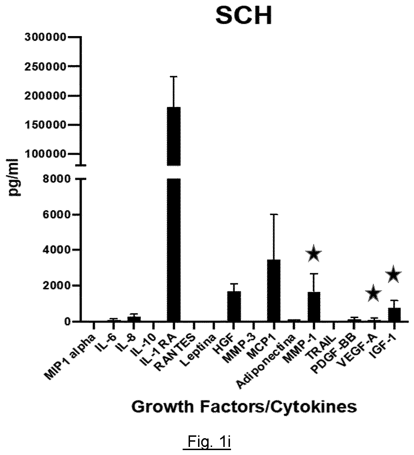

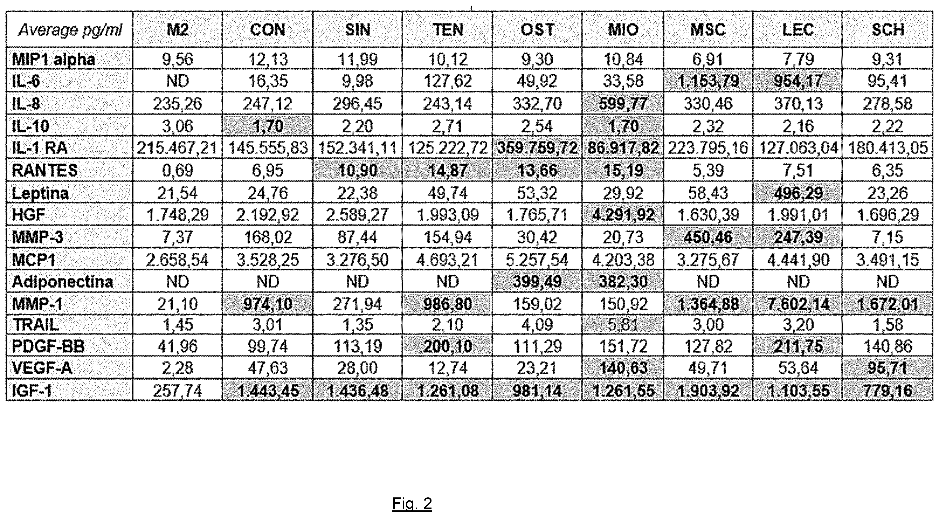

The results obtained in the multiplex assays were quantified in a Luminex LabScan 100 (Luminex), and those results from ELISA assays were measured using and iMark Microplate Absorbance Reader (Biorad). The measured levels from all samples and factors were interpolated using internal standard curves to obtain the concentration, and all results are expressed in pg/ml in **Figure 1** and **Figure 2****.**

The growth factors PDGF, TGF-β, IGF 1, VEGF and EGF are related to the cell migration and proliferation, playing an important role in the healing of muscular injuries. Moreover, IL-1 Beta and IL-6 are pro-inflammatory interleukins, whereas IL-10 is an anti-inflammatory interleukin. The balance among them modulates the inhibitory or stimulatory signals during the inflammation process, in an autocrine, paracrine and endocrine form.

The modification in the composition, based on the biochemical profile, of the medium conditioned by the anti-inflammatory macrophages M2 (M2-CM) provided by the described co-culture method is specific to each tissue, with specific molecular patterns or signatures (**Figure 3**) that optimize the natural regenerative process of each tissue. The patterns obtained show that the concentrations of the different molecules studied maintain the same relative proportions among them, and these proportions are specific for each tissue.

The tissue-specific compositions thereby obtained, maintain the typical anti-inflammatory properties of M2 macrophage secretome, such as high concentrations of IL-1 RA (IL-1β antagonist), but are modified to obtain a higher concentration of, for example, IGF-1 or HGF; the former, is elevated in all the different tissue-specific conditioned media, whereas the latter is significantly more concentrated only in the muscular tissue-specific conditioned medium (Fig. 2). Similarly, it can be observed how each modification of the M2-CM is specific for each tissue of interest, concentrating, decreasing or maintaining the relative levels of each cytokine or growth factor specifically (Fig. 3). Many other concentrations of molecules are modified in the products obtained by the described method, but always maintaining the same specific tissue patterns for the stated molecules. In fact, concentrated formulations either by centrifugation, lyophilization, drying, vacuum, purification columns or by any other method will retain the described modification patterns or tissue-specific composition signatures.

### Example 3: Clinical assays

Tissue-specific compositions obtained as described in Example 1 were tested in the treatment of injuries in muscles, tendons, ligaments, cartilages and bones in a total of 327 patients suffering from any of those injuries. Each composition was obtained by culturing M2-macrophages with cells of the same type as the target tissue. The tissue-specific compositions were frozen at -35 °C, cryopreserved at -86 °C, and thawed prior to their clinical use.

The patients practiced sport activities such as football, tennis, running, athletics, trekking, paddle and basketball. The age of the subjects was between 15 and 58 years old (average age 36 years old). 67 % of the patients were male and 33 % were female subjects.

The injuries of each case were classified according to the Rutland scale of patellar tendinopathy, as indicated in Table 1 (Rutland M *et al.,* 2010. N Am J Sports Phys Ther 5(3):166).

**Table 1. Phases of patellar tendinopathy**

| **Phases** | **Blazina jumper's knee Scale** | **Kennedy tendinopathy stages** |
|---|---|---|
| **Phase 1** | Pain after activity only | Pain after activity |
| **Phase 2** | Pain/discomfort during and after activity with the subject still able to perform at a satisfactory level (does not interfere with participation) | Pain at the beginning and after activity |
| **Phase 3** | Pain during and after activity with more prolonged, with subject having progressively increasing difficulty in performing at a satisfactory level (interferes with competition) | Pain at the beginning, during, and after activity, but the performance is not affected |
| **Phase 4** | Complete tendon disruption | Pain at the beginning, during and after activity, and the performance is affected |

The subjects treated in the assay were classified as indicated in Table 2 according to the level of injury for each case:

**Table 2. Classification of the patients according to the type and severity of the injury.**

| **Injury** | **Cases (N)** | **Phase 2** | **Phase 3** | **Phase 4** |
|---|---|---|---|---|
| Patellar tendinosis | 22 | 12 | 10 | 0 |
| Achilles tendinitis | 19 | 8 | 11 | 0 |
| Muscle rupture IT | 28 | 14 | 14 | 0 |
| Muscle rupture RA | 19 | 10 | 9 | 0 |
| Gonarthrosis | 71 | 45 | 26 | 0 |
| Chondromalacia patellae | 66 | 34 | 32 | 0 |
| Anterior cruciate ligament rupture | 14 | 8 | 6 | 0 |
| Dynamic osteopathy of the pubis | 38 | 18 | 12 | 8 |
| Astragalus fibular ligament sprain | 50 | 35 | 15 | 0 |

In order to assess the recovery in the subjects' tissue, the VISA score was used (Victoria Institute of Sport Assessment, Visentini PJ et al., 1998. J Sci Med Sport 1(1):22).

### Example 4: Treatment of patellar tendinosis

22 subjects suffering from patellar tendinosis (or tendinopathy) were selected. Patellar tendinosis is a musculoskeletal disorder affecting both recreational and elite athletes potentially leading to disability lasting several months. 12 of these subjects presented an initial phase II and 10, suffered an initial phase III. In order to assess the recovery in the subjects' tissue, the VISA score was used.

The composition, which was obtained by co-culturing with tenocytes, was injected into the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

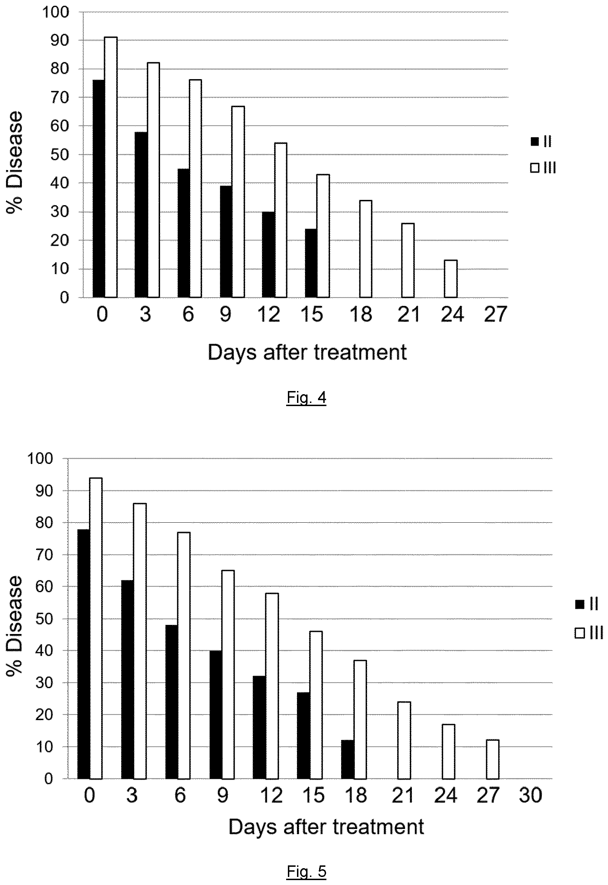

**Figure 4** shows the evolution of the percentage of scale of each phase of disease, from day 0 (control) until day 27.

Almost the 50 % of the patients with phase II injuries which were treated with the composition could return to the sport practice on day 3 after the treatment (PRS). On day 18, all these patients were performing sports. Moreover, the recovery time of the patients was even less than other treatments, such as PRS. After 27 days, all the patients with phase III injuries had returned to normal sport activity.

### Example 5: Treatment of Achilles tendinitis

19 subjects suffering from patellar tendinosis (or tendinopathy) were selected. Patellar tendinosis is a musculoskeletal disorder affecting both recreational and elite athletes potentially leading to disability lasting several months. 8 of these subjects presented an initial phase II and 11, suffered an initial phase III.

The composition, which was obtained by co-culturing with tenocytes, was injected peri-tendon and intra-tendon in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

**Figure 5** shows the evolution of the percentage of scale of each phase of disease, from day 0 (control) until day 30. On day 6, more than the 50 % of the patients with phase II injuries which were treated with the composition could return to the sport practice after the treatment (PRS).

### Example 6: Treatment of muscle tear

47 subjects suffering from muscle tear were selected. 28 of the subjects suffered hamstrings and 19 suffered a rupture of the quadriceps anterior rectum. 24 of the subjects presented an initial phase II and 23, suffered an initial phase III. In order to evaluate the effect of the composition in each subject, it was evaluated the percentage of players who got back to sports activity (RTP) after the treatment.

The composition, which was obtained by co-culturing with myocytes, was injected peri-tendon and intra-tendon in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

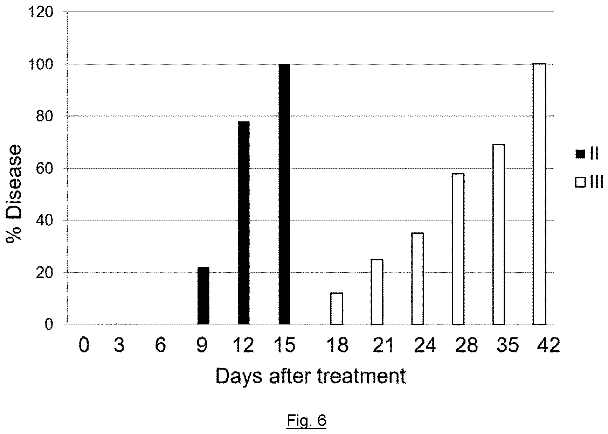

**Figure 6** shows the result of the treatment with the percentage of patients which returned to sport activity in a specific day. In grade II injuries, more than 50% of patients treated with PRS returned to sports on the 10th day after the treatment, 100% being on the 15th day after completion of the treatment.

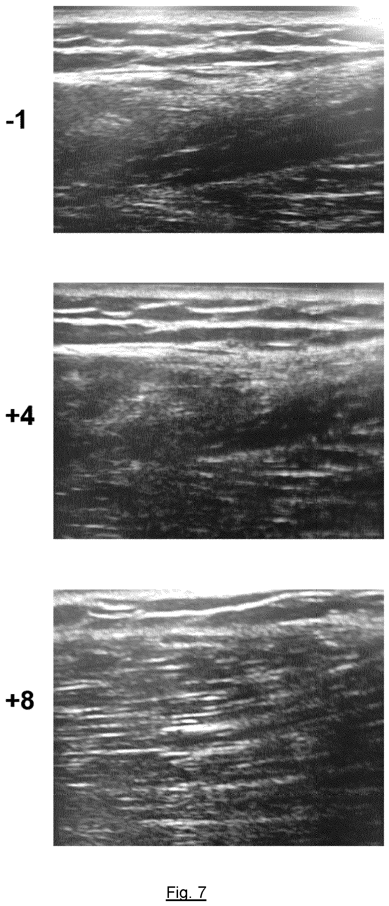

In order to evaluate the progression of the recovery of the injury, a follow-up of one of the patients, a 36 years old male with a fibrillar tear lower third of the biceps femoris, performed, in order to evaluate the healing of the muscle. **Figure 7** shows the result of an echography done on the day previous to the treatment (-1), the day 4 after the treatment (+4) and the day 8 after the treatment (+8).

From these results, it may be concluded that the patients to who the composition was applied, improved during the treatment, recovering from the injuries.

### Example 7: Treatment of gonarthrosis

71 subjects suffering from gonarthrosis were selected. 45 of the subjects presented an initial phase II and 26, suffered an initial phase III. In order to evaluate the effect of the composition in each subject, it was evaluated the functional and knee pain scale WOMAC scale (The Western Ontario and McMaster Universities Osteoarthritis Index).

The composition, which was obtained by co-culturing with chondrocytes, was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

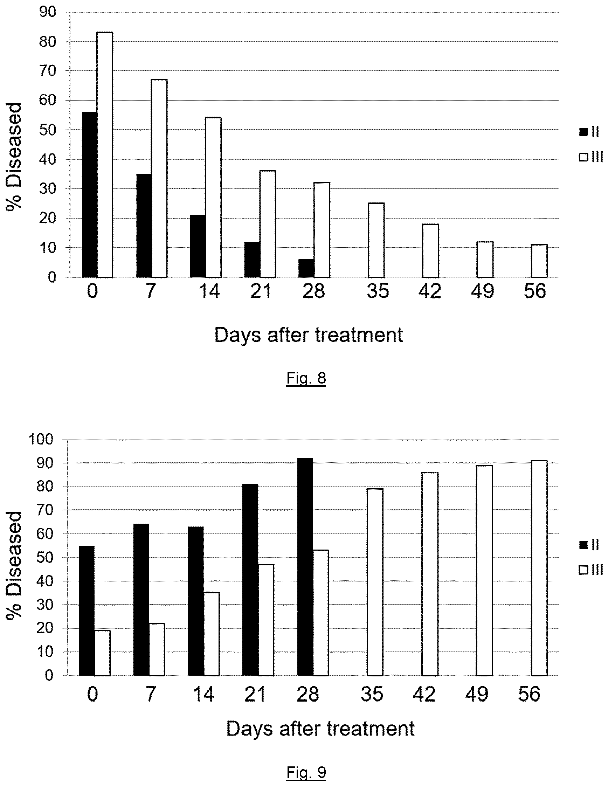

**Figure 8** shows the result of the treatment with the percentage of patients which returned to sport activity in a specific day. In grade II injuries, more than 60% of patients treated with PRS returned to sports on the 7th day after the treatment, 100% being on the 35th day after completion of the treatment.

From these results, it may be concluded that the patients to who the composition was applied, improved during the treatment, recovering from the injuries.

### Example 8: Treatment of chondromalacia patellae

66 subjects suffering from gonarthrosis were selected. 34 of the subjects presented an initial phase II and 32, suffered an initial phase III. In order to evaluate the effect of the composition in each subject, it was evaluated the international KOOS scale for the function and knee pain evaluation (Knee injury and Osteoarthritis Outcome Score.

The composition, which was obtained by co-culturing with chondrocytes, was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

Figure 9 shows the result of the treatment with the percentage of patients which returned to sport activity in a specific day (RTP, return to play).

### Example 9: Treatment of anterior cruciate ligament rupture (ACL)

14 subjects suffering from gonarthrosis were selected. 8 of the subjects presented an initial phase II and 6, suffered an initial phase III. In order to evaluate the effect of the composition in each subject, it was evaluated the KOOS scale. The phase III case were under semitendinosus plastic surgery.

The composition, which was obtained by co-culturing with tenocytes, was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

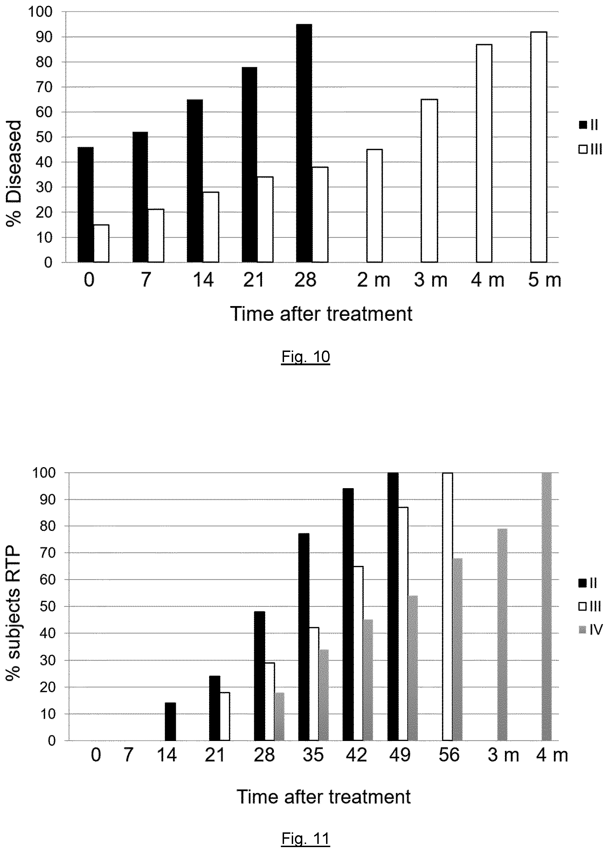

**Figure 10** shows the result of the treatment with the percentage of patients which returned to sport activity in a specific day.

### Example 10: Treatment of dynamic osteopathy of the pubis (DOC)

38 subjects suffering from DOC were selected. 18 of the subjects presented an initial phase II, 12, suffered an initial phase III and 8 an initial phase IV. In order to evaluate the effect of the composition in each subject, it was evaluated the percentage of players who got back to sports activity (RTP) after the treatment.

The composition, which was obtained by co-culturing with chondroblasts, was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

**Figure 11** shows the result of the treatment with the percentage of patients which returned to sport activity in a specific day.

### Example 11: Treatment of astragalus fibular ligament sprain (AFL)

50 subjects suffering from LPA were selected. 35 of the subjects presented an initial phase II and 15, suffered an initial phase III. In order to evaluate the effect of the composition in each subject, it was evaluated the FAAM scale (Foot and Ankle Ability Measure.

The composition, which was obtained by co-culturing with osteocytes, was injected intra-articular in aseptic conditions in the patellar tendon of the subjects. The patients were followed up at 10 days, 1 month, 3 months and 6 months after the injection of the composition, in which the VISA scale data were evaluated. In addition, previous and post-treatment ultrasounds were performed 10 days after implantation.

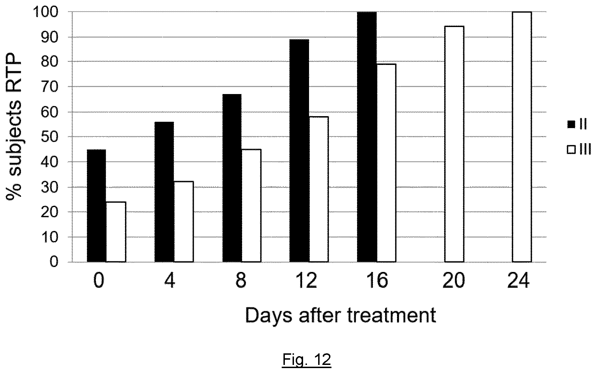

**Figure 12** shows the result of the treatment with the percentage of patients which returned to sport activity in a specific day.

## Claims

1. Method for obtaining a composition for tissue regeneration, comprising the following steps:
- providing M2-macrophages, wherein this step of providing M2-macrophages comprises purifying monocytes from a sample and differentiating the purified monocytes into M2-macrophages;
- co-culturing the M2-macrophages with tissue-specific cells in serum free medium; and
- collecting the supernatant of the co-culture,
wherein the ratio of M2-macrophages to tissue-specific cells in the co-culture is 1:1 to 40:1.

2. The method of claim 1, wherein the M2-macrophages are co-cultured with the tissue-specific cells at a temperature of between 30 and 40°C.

3. The method of claim 1 or 2, wherein the M2-macrophages are co-cultured with the tissue-specific cells for a period of 3 to 28 days.

4. The method of any of the preceding claims, wherein the ratio of M2-macrophages to tissue-specific cells in the co-culture is 1:1 to 20:1.

5. The method of any of the preceding claims, wherein the co-culture is carried out in hypoxia.

6. The method of claim 1, **characterized in that** the step of differentiating the purified monocytes into M2-macrophages comprises culturing the monocytes in serum free medium in presence of macrophage colony-stimulating factor (M-CSF).

7. The method of claim 6, **characterized in that** the step of differentiating the purified monocytes into M2-macrophages comprises culturing the monocytes in serum free medium in presence of macrophage colony-stimulating factor (M-CSF) for 3-5 days.

8. The method of claims 1 to 7, wherein the monocytes are autologous or allogenic.

9. The method of any of the preceding claims, further comprising the steps of cryopreservation or lyophilization of the collected supernatant.

10. The method of any of the preceding claims, wherein the tissue-specific cells are cells corresponding to the tissue to be regenerated.

11. The method of claim 10, wherein the tissue-specific cells are autologous or allogenic.

12. The method of any of claims 1 to 11, wherein the tissue-specific cells are selected from the group comprising myocytes, skeletal muscle cells, muscle satellite cells, tenocytes, osteocytes, Schwann cells, dermal fibroblasts, chondrocytes, synoviocytes, lymphocytes, hair follicle cells, myocardia cells, cells from respiratory epithelium, lymphatic endothelial cells or mesenchymal stem cells or combinations thereof.

13. The method of any one of claims 1 to 12, wherein the step of co-culturing the M2-macrophages with tissue-specific cells in serum free medium is carried out in a Transwell.

14. Composition for tissue regeneration obtained by a method consisting of the following steps:
- providing M2-macrophages, wherein this step of providing M2-macrophages comprises purifying monocytes from a sample and differentiating the purified monocytes into M2-macrophages;
- co-culturing the M2-macrophages with tissue-specific cells in serum free medium; and
- collecting the supernatant of the co-culture,
wherein the ratio of M2-macrophages to tissue-specific cells in the co-culture is 1:1 to 40:1, wherein the tissue-specific cells are selected from the group comprising myocytes, skeletal muscle cells, muscle satellite cells, tenocytes, osteocytes, Schwann cells, dermal fibroblasts, chondrocytes, synoviocytes, lymphocytes, hair follicle cells, myocardia cells, cells from respiratory epithelium, lymphatic endothelial cells or mesenchymal stem cells or combinations thereof and wherein the M2-macrophages are co-cultured with the tissue-specific cells for a period of 3 to 28 days.

15. The composition of claim 14, wherein the step of co-culturing the M2-macrophages with tissue-specific cells in serum free medium is carried out in a Transwell.

16. The composition of claim 14 or claim 15, wherein the tissue-specific cells are selected from the group comprising tenocytes, mesenchymal stem cells, myocytes, chondrocytes, Schwann cells, synoviocytes, lymphatic endothelial cells or osteoblasts.

17. The composition according to any of claims 14 to 16, wherein the total amount of platelets, red cells or leukocytes in the composition are less than 0.1 % of the total amount of components in the sample.

18. The composition according to any of claims 14 to 17, wherein it comprises platelets, red cells or leukocytes in an amount of less than 0.001% of the total amount of components in the sample.

19. Pharmaceutical composition comprising the composition of claims 14 to 18 and a pharmaceutically acceptable carrier.

20. A composition in accordance with any one of claims 14 to 18 for use in a method of treatment of a disease, wherein the composition is administered in a tissue comprising cells of the same type as the tissue-specific cells.

21. The composition for use according to claim 20, wherein the disease is selected from tendinosis, muscle fibrillary break, tendinitis, bursitis, synovitis, arthrosis, cartilage break, respiratory disease, cardiac disease or chondropathies.

22. The composition for use according to any of claims 20 or 21, wherein the disease is selected from patellar tendinosis, partial muscular ruptures, Achilles tendonitis, gonarthrosis, patellar chondropathy, anterior cruciate ligament tear, dynamic pubic osteopathy or sprained astragalus ligament.

23. The composition for use according to claims 20 to 22, wherein the composition is administered to a subject different from the subject donor of the monocytes or the tissue-specific cells.

24. The composition for use according to claims 20 to 23, wherein the composition is cryopreserved or lyophilized.

25. The composition for use according to claims 21 to 24, wherein the composition is administered in a tissue comprising cells of the same type as the tissue-specific cells.

26. Method for modifying the growth factor and/or cytokine composition of the culture medium in a M2-macrophage culture, comprising:
- providing M2-macrophages, wherein this step of providing M2-macrophages comprises purifying monocytes from a sample and differentiating the purified monocytes into M2-macrophages; and
- co-culturing the M2-macrophages with tissue-specific cells in serum free medium,
wherein the growth factor and/or cytokine composition of the culture medium is modified as compared to the growth factor and/or cytokine composition of a culture medium in a M2-macrophage culture without the tissue-specific cells in serum free medium, wherein the ratio of M2-macrophages to tissue-specific cells in the co-culture is 1:1 to 40:1.

## Patentansprüche

1. Verfahren zur Herstellung einer Zusammensetzung zur Geweberegeneration, das die folgenden Schritte umfasst:
- Bereitstellen von M2-Makrophagen, wobei dieser Schritt des Bereitstellens von M2-Makrophagen das Reinigen von Monozyten aus einer Probe und das Differenzieren der gereinigten Monozyten in M2-Makrophagen umfasst;
- gemeinsames Kultivieren der M2-Makrophagen mit gewebespezifischen Zellen in serumfreiem Medium; und
- Entnahme des Überstands der gemeinsamen Kultur,
wobei das Verhältnis von M2-Makrophagen zu gewebespezifischen Zellen in der gemeinsamen Kultur 1:1 bis 40:1 beträgt.

2. Verfahren nach Anspruch 1, wobei das gemeinsame Kultivieren der M2-Makrophagen mit den gewebespezifischen Zellen bei einer Temperatur zwischen 30 und 40 °C erfolgt.

3. Verfahren nach Anspruch 1 oder 2, wobei das gemeinsame Kultivieren der M2-Makrophagen mit den gewebespezifischen Zellen über einen Zeitraum von 3 bis 28 Tagen erfolgt.

4. Verfahren nach einem der vorstehenden Ansprüche, wobei das Verhältnis von M2-Makrophagen zu gewebespezifischen Zellen in der gemeinsamen Kultur 1:1 bis 20:1 beträgt.

5. Verfahren nach einem der vorstehenden Ansprüche, wobei das gemeinsame Kultivieren unter Hypoxie erfolgt.

6. Verfahren nach Anspruch 1, **dadurch gekennzeichnet, dass** der Schritt des Differenzierens der gereinigten Monozyten in M2-Makrophagen das Kultivieren der Monozyten in serumfreiem Medium unter Vorhandensein von Makrophagen-Kolonie-stimulierendem Faktor (M-CSF) umfasst.

7. Verfahren nach Anspruch 6, **dadurch gekennzeichnet, dass** der Schritt des Differenzierens der gereinigten Monozyten in M2-Makrophagen das Kultivieren der Monozyten in serumfreiem Medium unter Vorhandensein von Makrophagen-Kolonie-stimulierendem Faktor (M-CSF) für 3 - 5 Tage umfasst.

8. Verfahren nach einem der Ansprüche 1 bis 7, wobei die Monozyten autolog oder allogen sind.

9. Verfahren nach einem der vorstehenden Ansprüche, das ferner die Schritte der Kryokonservierung oder Gefriertrocknung des entnommenen Überstands umfasst.

10. Verfahren nach einem der vorstehenden Ansprüche, wobei die gewebespezifischen Zellen mit den Zellen des zu regenerierenden Gewebes übereinstimmen.

11. Verfahren nach Anspruch 10, wobei die gewebespezifischen Zellen autolog oder allogen sind.

12. Verfahren nach einem der Ansprüche 1 bis 11, wobei die gewebespezifischen Zellen aus der Gruppe bestehend aus Myozyten, Skelettmuskelzellen, Muskelsatellitenzellen, Tenozyten, Osteozyten, Schwann-Zellen, dermale Fibroblasten, Chondrozyten, Synoviozyten, Lymphozyten, Haarfollikelzellen, Herzmuskelzellen, Zellen des Atemwegsepithels, lymphatische Endothelzellen, mesenchymale Stammzellen oder Kombinationen davon ausgewählt sind.

13. Verfahren nach einem der Ansprüche 1 bis 12, wobei der Schritt des gemeinsamen Kultivierens der M2-Makrophagen mit gewebespezifischen Zellen in serumfreiem Medium in einem Transwell erfolgt.

14. Zusammensetzung zur Geweberegeneration, hergestellt durch ein aus folgenden Schritten bestehendes Verfahren:
- Bereitstellen von M2-Makrophagen, wobei dieser Schritt des Bereitstellens von M2-Makrophagen das Reinigen von Monozyten aus einer Probe und das Differenzieren der gereinigten Monozyten in M2-Makrophagen umfasst;
- gemeinsames Kultivieren der M2-Makrophagen mit gewebespezifischen Zellen in serumfreiem Medium; und
- Entnahme des Überstands der gemeinsamen Kultur,
wobei das Verhältnis von M2-Makrophagen zu gewebespezifischen Zellen in der gemeinsamen Kultur 1:1 bis 40:1 beträgt, wobei die gewebespezifischen Zellen aus der Gruppe bestehend aus Myozyten, Skelettmuskelzellen, Muskelsatellitenzellen, Tenozyten, Osteozyten, Schwann-Zellen, dermale Fibroblasten, Chondrozyten, Synoviozyten, Lymphozyten, Haarfollikelzellen, Herzmuskelzellen, Zellen des Atemwegsepithels, lymphatische Endothelzellen, mesenchymale Stammzellen oder Kombinationen davon ausgewählt sind und wobei das gemeinsame Kultivieren der M2-Makrophagen mit den gewebespezifischen Zellen über einen Zeitraum von 3 bis 28 Tagen erfolgt.

15. Zusammensetzung nach Anspruch 14, wobei der Schritt des gemeinsamen Kultivierens der M2-Makrophagen mit gewebespezifischen Zellen in serumfreiem Medium in einem Transwell erfolgt.

16. Zusammensetzung nach Anspruch 14 oder 15, wobei die gewebespezifischen Zellen aus der Gruppe bestehend aus Tenozyten, mesenchymale Stammzellen, Myozyten, Chondrozyten, Schwann-Zellen, Synoviozyten, lymphatische Endothelzellen oder Osteoblasten ausgewählt sind.

17. Zusammensetzung nach einem der Ansprüche 14 bis 16, wobei die Gesamtmenge der Thrombozyten, Erythrozyten oder Leukozyten in der Zusammensetzung weniger als 0,1 % der Gesamtmenge der Komponenten in der Probe beträgt.

18. Zusammensetzung nach einem der Ansprüche 14 bis 17, wobei sie Thrombozyten, Erythrozyten oder Leukozyten in einer Menge von weniger als 0,001 % der Gesamtmenge der Komponenten in der Probe enthält.

19. Pharmazeutische Zusammensetzung, umfassend die Zusammensetzung nach einem der Ansprüche 14 bis 18 und einen pharmazeutisch verträglichen Träger.

20. Zusammensetzung nach einem der Ansprüche 14 bis 18 zur Verwendung in einem Verfahren zur Behandlung einer Erkrankung, wobei die Anwendung der Zusammensetzung in einem Gewebe erfolgt, das die gleiche Art von Zellen wie die gewebespezifischen Zellen enthält.

21. Zusammensetzung zur Verwendung nach Anspruch 20, wobei die Erkrankung ausgewählt ist aus Tendinose, Muskelfaserriss, Tendinitis, Bursitis, Synovitis, Arthrose, Knorpelriss, Atemwegserkrankungen, Herzerkrankungen oder Chondropathien.

22. Zusammensetzung zur Verwendung nach einem der Ansprüche 20 oder 21, wobei die Erkrankung ausgewählt ist aus Tendinose der Patellasehne, Muskelteilrissen, Tendinose der Achillessehne, Gonarthrose, Chondropathia patellae, Riss des vorderen Kreuzbandes, Osteitis pubis oder Zerrung des Sprungbeinbands.

23. Zusammensetzung zur Verwendung nach einem der Ansprüche 20 bis 22, wobei die Anwendung der Zusammensetzung bei einer Person erfolgt, die nicht der Spender der Monozyten oder der gewebespezifischen Zellen ist.

24. Zusammensetzung zur Verwendung nach einem der Ansprüche 20 bis 23, wobei die Zusammensetzung kryokonserviert oder gefriergetrocknet wird.

25. Zusammensetzung zur Verwendung nach einem der Ansprüche 21 bis 24, wobei die Anwendung der Zusammensetzung in einem Gewebe erfolgt, das die gleiche Art von Zellen wie die gewebespezifischen Zellen enthält.

26. Verfahren zur Modifizierung von Wachstumsfaktor- und/oder Zytokinzusammensetzung des Kulturmediums in einer M2-Makrophagenkultur, das Folgendes umfasst:
- Bereitstellen von M2-Makrophagen, wobei dieser Schritt des Bereitstellens von M2-Makrophagen das Reinigen von Monozyten aus einer Probe und das Differenzieren der gereinigten Monozyten in M2-Makrophagen umfasst; und
- gemeinsames Kultivieren der M2-Makrophagen mit gewebespezifischen Zellen in serumfreiem Medium,
wobei die Wachstumsfaktor- und/oder Zytokinzusammensetzung des Kulturmediums im Vergleich zu der Wachstumsfaktor- und/oder Zytokinzusammensetzung eines Kulturmediums in einer M2-Makrophagenkultur ohne die gewebespezifischen Zellen in serumfreiem Medium modifiziert ist, wobei das Verhältnis von M2-Makrophagen zu gewebespezifischen Zellen in der gemeinsamen Kultur 1:1 bis 40:1 beträgt.

## Revendications

1. Procédé d'obtention d'une composition pour la régénération de tissus, comprenant les étapes suivantes :
- fournir des macrophages M2, dans lequel cette étape de fournir des macrophages M2 comprend purifier des monocytes à partir d'un échantillon et différencier les monocytes purifiés pour donner des macrophages M2 ;
- co-cultiver les macrophages M2 avec des cellules spécifiques de tissu dans un milieu sans sérum ; et
- récolter le surnageant de la co-culture,
dans lequel le rapport des macrophages M2 par rapport aux cellules spécifiques de tissu dans la co-culture est de 1:1 à 40:1.

2. Procédé selon la revendication 1, dans lequel les macrophages M2 sont co-cultivés avec les cellules spécifiques de tissu à une température d'entre 30 et 40°C.

3. Procédé selon la revendication 1 ou 2, dans lequel les macrophages M2 sont co-cultivés avec les cellules spécifiques de tissu pendant une période de 3 à 28 jours.

4. Procédé selon l'une quelconque des revendications précédentes, dans lequel le rapport de macrophages M2 par rapport aux cellules spécifiques de tissu dans la co-culture est de 1:1 à 20:1.

5. Procédé selon l'une quelconque des revendications précédentes, dans lequel la co-culture est réalisée en hypoxie.

6. Procédé selon la revendication 1, **caractérisé en ce que** l'étape de différencier les monocytes purifiés pour donner des macrophages M2 comprend la culture des monocytes dans un milieu sans sérum en présence de facteur de stimulation des colonies de macrophages (M-CSF).

7. Procédé selon la revendication 6, **caractérisé en ce que** l'étape de différencier les monocytes purifier pour donner des macrophages M2 comprend la culture des monocytes dans un milieu sans sérum en présence de facteur de stimulation des colonies de macrophage (M-CSF) pendant 3-5 jours.

8. Procédé selon les revendications 1 à 7, dans lequel les monocytes sont autologues ou allogéniques.

9. Procédé selon l'une quelconque des revendications précédentes, comprenant en outre les étapes de cryoconservation ou lyophilization du surnageant récolté.

10. Procédé selon l'une quelconque des revendications précédentes, dans lequel les cellules spécifiques de tissu sont des cellules correspondants au tissu à régénérer.

11. Procédé selon la revendication 10, dans lequel les cellules spécifiques de tissu sont autologues ou allogéniques.

12. Procédé selon l'un quelconque des revendications 1 à 11, dans lequel les cellules spécifiques de tissu sont choisies parmi le groupe comprenant les myocytes, les cellules des muscles squelettiques, les cellules satellites musculaires, les ténocytes, les ostéocytes, les cellules de Schwann, les fibroblastes dermiques, les chondrocytes, les synoviocytes, les lymphocytes, les cellules des follicules pileux, les cellules du myocarde, les cellules de l'épithélium respiratoire, les cellules endothéliales lymphatiques ou les cellules souches mésenchymateuses ou des combinaisons de celles-ci.

13. Procédé selon l'un quelconque des revendications 1 à 12, dans lequel l'étape de co-cultiver les macrophages M2 avec des cellules spécifiques de tissu dans un milieu sans sérum est réalisée dans un insert Transwell.

14. Composition pour la régénération de tissus obtenue par un procédé consistant en les étapes suivantes :
- fournir des macrophages M2, dans lequel cette étape de fournir des macrophages M2 comprend purifier des monocytes à partir d'un échantillon et différencier les monocytes purifiés pour donner des macrophages M2 ;
- co-cultiver les macrophages M2 avec des cellules spécifiques de tissu dans un milieu sans sérum ; et
- récolter les surnageant de la co-culture,
dans laquelle le rapport de macrophages M2 par rapport aux cellules spécifiques de tissu dans la co-culture est de 1:1 à 40:1, dans laquelle les cellules spécifiques de tissu sont choisies parmi le groupe comprenant les myocytes, les cellules des muscles squelettiques, les cellules satellites musculaires, les ténocytes, les ostéocytes, les cellules de Schwann, les fibroblastes dermiques, les chondrocytes, les synoviocytes, les lymphocytes, les cellules des follicules pileux, les cellules du myocarde, les cellules de l'épithélium respiratoire, les cellules endothéliales lymphatiques ou les cellules souches mésenchymateuses ou des combinaisons de celles-ci, et dans laquelle les macrophages M2 sont co-cultivés avec les cellules spécifiques de tissu pendant une période de 3 à 28 jours.

15. Composition selon la revendication 14, dans laquelle l'étape de co-cultiver les macrophages M2 avec des cellules spécifiques de tissu dans un milieu sans sérum est réalisée dans un insert Transwell.

16. Composition selon la revendication 14 ou la revendication 15, dans laquelle les cellules spécifiques de tissu sont choisies parmi le groupe comprenant les ténocytes, les cellules souches mésenchymateuses, les myocytes, les chondrocytes, les cellules de Schwann, les synoviocytes, les cellules endothéliales lymphatiques ou les ostéoblastes.

17. Composition selon l'une quelconque des revendications 14 à 16, dans laquelle la quantité totale de plaquettes, de globules rouges ou de leucocytes dans la composition sont inférieures à 0,1% de la quantité totale de composants dans l'échantillon.

18. Composition selon l'une quelconque des revendications 14 à 17, dans laquelle elle comprend des plaquettes, des globules rouges ou des leucocytes dans une quantité de moins de 0,001% de la quantité totale de composants dans l'échantillon.

19. Composition pharmaceutique comprenant la composition selon les revendications 14 à 18 et un véhicule pharmaceutiquement acceptable.

20. Composition selon l'une quelconque des revendications 14 à 18 pour son utilisation dans un procédé de traitement d'une maladie, dans laquelle la composition est administrée dans un tissu comprenant des cellules du même type que les cellules spécifiques de tissu.

21. Composition pour son utilisation selon la revendication 20, dans laquelle la maladie est choisie parmi la tendinose, la rupture fibrillaire musculaire, la tendinite, la bursite, la synovite, l'arthrose, la rupture de cartilage, les maladies respiratoires, les maladies cardiaques ou les chondropathies.

22. Composition pour son utilisation selon l'une quelconque des revendications 20 ou 21, dans laquelle la maladie est choisie parmi la tendinose rotulienne, les ruptures musculaires partielles, la tendinite d'Achilles, la gonarthrose, la chondropathie rotulienne, la déchirure du ligament croisé antérieur, l'ostéopathie pubienne dynamique ou l'entorse du ligament astragale.

23. Composition pour son utilisation selon les revendications 20 à 22, dans laquelle la composition est administrée à un sujet différent du sujet donneur des monocytes ou des cellules spécifiques de tissu.

24. Composition pour son utilisation selon les revendications 20 à 23, dans laquelle la composition est cryoconservée ou lyophilisée.

25. Composition pour son utilisation selon les revendications 21 à 24, dans laquelle la composition est administrée dans un tissu comprenant des cellules du même type que les cellules spécifiques de tissu.

26. Procédé pour modifier la composition en facteurs de croissance et/ou en cytokines du milieu de culture dans une culture de macrophages M2, comprenant :
- fournir des macrophages M2, dans lequel cette étape de fournir des macrophages M2 comprend purifier des monocytes à partir d'un échantillon et différencier les monocytes purifiés pour donner des macrophages M2 ; et
- co-cultiver les macrophages M2 avec des cellules spécifiques de tissu dans un milieu sans sérum,
dans lequel la composition en facteurs de croissance et/ou en cytokines du milieu de culture est modifiée par rapport à la composition en facteurs de croissance et/ou en cytokines d'un milieu de culture dans une culture de macrophages M2 sans les cellules spécifiques de tissu dans un milieu sans sérum, dans lequel le rapport de macrophages M2 par rapport aux cellules spécifiques de tissu dans la co-culture est de 1:1 à 40:1.
